# EUROPEAN PATENT APPLICATION

(11) **EP 2 389 908 A1**
(43) Date of publication of application: **30.11.2011**
(21) Application number: 09838739.2
(22) Date of filing: 04.12.2009
(51) Int. Cl.: A61F 5/441, A61F 5/44, A61F 5/451

(54) **AUTOMATIC URINE COLLECTING DEVICE**

(30) Priority: 20.01.2009 JP 2009009776
(71) Applicant: Hitachi, Ltd., Chiyoda-ku Tokyo 100-8280 (JP)
(72) Inventor: TANAKA, Tetsuya, Tokyo 101-8608 (JP); MIYAGAWA, Ryosuke, Tokyo 101-8608 (JP); ISHITSUKA, Yoshikazu, Tokyo 101-8608 (JP); SAYAMA, Shigeharu, Tagawa-gun Fukuoka 822-1296 (JP)
(74) Representative: Beetz & Partner
(86) International application number: PCT/JP2009/006635
(87) International publication number: WO 2010/084545

(57) **Abstract**

An automatic urine collecting device (1) is provided with: a main tank (20) for collecting and containing urine received by a urine receiver; a body case for supporting the main tank (20) ; a suction pump (53) for sucking the urine, which is received by the urine receiver, into the main tank (20) through a urine flow path by sucking air within the main tank through an airflow path; a urine sensor for detecting urine; and a control device for outputting a control command on the basis of the result of the detection by the urine sensor. The urine sensor is provided with a main-tank infrared sensor (S31) capable of detecting bubbles present in the upper part of the urine contained in the main tank (20). The configuration enhances the reliability of the suction pump.

## Description

### Technical Field

The present invention relates to an automatic urine collecting device and in particular is preferable for an automatic urine collecting device for automatically collecting urine excreted by bedridden patients, old people, etc.

### Background Art

In recent years, automatic urine collecting systems for automatically collecting urine excreted bedridden patients, old people, etc. are known. Typically the automatic urine collecting system mainly includes a urine receiver installed to a part of, e.g., a patient via a diaper etc., a tube having one end connected to the urine receiver, and an automatic urine collecting device connected to the other end of this tube. As the automatic urine collecting device, one including a collection container for containing urine discharged from a urine receiver through a tube and a suction pump for sucking the urine contained in the urine receiver and carrying the urine to the collection container is conventionally known.

As this conventional type of device, there is, e.g., a urine suction device disclosed in JP-A No. 2003-126242 (Patent Document 1). The urine suction device of Patent Document 1 includes a suction pump for sucking urine from a urine receiver through a suction pipeline, a detector for detecting the upper limit amount of urine which can be contained in a urine collection container, and a control device for controlling the suction pump. The detector includes a float including therein a permanent magnet and a magnetic sensor. The float is floated in urine in a urine containing portion. The magnetic sensor is provided to the upper surface of a lid of the urine containing portion. When urine reaches the maximum level, an output is generated from the magnetic sensor. The control device provides controls for stopping the suction pump and generating an alarm on the basis of a signal outputted from the magnetic sensor when an amount of the urine in the urine collection container reaches the upper limit.

As a conventional automatic urine collecting device, there is , for example, one disclosed in JP-A No. 2008-5975 (Patent Document 2). The automatic urine collecting device of the Patent Document 2 includes a collection container for containing urine sent from a urine receiver through a urine flow path and a body part for supporting the collection container. The body part includes: a suction pump for sucking urine received by the urine receiver through a urine flow path by sucking air in the collection container through an airflow path and for carrying the urine to the collection container; a mass sensor for measuring a mass of the urine contained in the collection container; a control substrate for calculating a capacity of urine on the basis of the measured mass; and display means for displaying a capacity of the calculated urine. When the control substrate determines that the container is close to the full state (for example, 60% or more than the effective capacity of the collection container) as a result of the determination of the urine amount, the control substrate alarms a user by providing, e.g., a control for flashing a lamp of a display part. Further, when the control substrate determines that the container is in the full state (for example, the effective capacity of the collection container) as a result of the determination of the urine amount, the control substrate sounds an alarm by use of alarm means while transmitting a stop signal to the suction pump.

### Related Art Documents

### Patent Documents

Patent Document 1: JP-A No. 2003-126242
Patent Document 2: JP-A No. 2008-5975

### Summary of the Invention

### Problem to be solved by the invention

In the urine suction device of Patent Document 1 mentioned above, bubbles present in the upper part of the urine contained in the urine collection container are not considered. Urine sucked through a suction pipeline from the urine receiver are associated with many bubbles. Bubbles are formed on the upper part of the urine contained when urine is collected and contained in the urine collection container. Typically, the float is difficult to float by the bubbles. In the urine suction device of Patent Document 1, before a liquid level of urine rises and reaches the maximum level, bubbles on the upper part of the urine reaches an air inlet of the suction pipeline (airflow path for sucking air in the urine collection container) and may be sucked by the suction pump through the suction pipeline from the urine collection container. This may cause failure of the suction pump.

In the automatic urine collecting device of Patent Document 2 mentioned above, bubbles present in the upper part of the urine contained in the collection container is not considered. The mass sensor is typically difficult to measure the existence and height of bubbles. Therefore, in the automatic urine collecting device of Patent Document 2, before determining that a urine amount is in the full state, bubbles on the upper part of the urine reaches an air inlet of the airflow path and may be sucked by the suction pump from the collection container through the airflow path. This may cause failure of the suction pump.

An object of the present invention is to provide an automatic urine collecting device capable of improving reliability of a suction pump.

### Means for solving the Problem

In the first aspect of the present invention for achieving the above-mentioned object, an automatic urine collecting device comprises: a main tank for collecting and containing urine received by a urine receiver; a body case for supporting the main tank; a suction pump for sucking the urine received by the urine receiver into the main tank through a urine flow path by sucking air within the main tank through an airflow path; a urine sensor for detecting urine; and a control device for outputting a control command on the basis of the detection result of the urine sensor. In the automatic urine collecting device, the urine sensor includes a main tank infrared sensor capable of detecting bubbles present in the upper part of the urine contained in the main tank.

Preferred concrete structures in the first aspect of the present invention are as follows.
(1) Notification means for warning of discard of urine contained in the main tank is provided, the main tank is installed in the body case detachably, and the control device controls the notification means on the basis of the detection result of the main-tank infrared sensor.
(2) In the above (1), the body case includes an operation panel on which a control switch and a display lamp are arranged, the notification means includes the display lamps for warning by flashing and an alarm by sound, and the control switch includes a temporal muting switch for stopping operation of the alarm.
(3) The body case has a tank housing recess, the main tank is installed in the tank housing recess detachably and is formed of a translucent resin material transparent to infrared light, and the main-tank infrared sensor includes a light emitting part and a light receiving part respectively arranged on both sides of the main tank to oppose to one another via the main tank.
(4) The urine sensor includes the main-tank infrared sensor and a main-tank liquid level sensor for detecting a liquid level of urine in the main tank.
(5) In the above (4), the main-tank liquid level sensor includes: a float floating when a liquid level of urine in the main tank rises; a ball valve for closing an air inlet of the airflow path by the floating of the float; and detection means for detecting a current change of a driving motor of the suction pump.
(6) In the above (4) or (5), notification means for warning of discard of urine contained in the main tank is provided, the main tank is installed to the body case detachably, and the control device controls the notification means on the basis of the detection result of the main-tank infrared sensor and stops the operation of the suction pump or controls the notification means to warn on the basis of the result of the detection by the main-tank liquid level sensor.
(7) A sub tank for containing urine which has flowed into the airflow path is provided in the middle of the airflow path.
(8) In the above (7), the urine sensor includes the main-tank infrared sensor and a sub-tank sensor for detecting urine in the sub tank.
(9) In the above (8), the sub-tank sensor includes a sub-tank infrared sensor capable of detecting bubbles which have flowed into the sub tank.
(10) In the above (9), the sub tank is formed of a translucent resin material transparent to infrared light, and the sub-tank infrared sensor includes a light emitting part and a light receiving part respectively arranged on both sides of the sub tank to oppose to one another via the sub tank.
(11) In the above (8), notification means for warning of discard of urine contained in the main tank is provided, the main tank is installed in the body case detachably, and the control device controls the notification means to warn on the basis of the detection result of the main-tank infrared sensor and stops the operation of the suction pump on the basis of the detection result of the sub-tank sensor.
(12) The main tank includes a main-tank body for containing urine and a main-tank lid installed to the center of the upper surface of the main-tank body detachably and having openings forming part of the urine flow path and part of the airflow path adjacently, and the urine flow path includes a T-shaped discharge pipe branching horizontally within the main tank to be opened oppositely.
(13) An anti-bubble net is provided to cover the air inlet of the airflow path opened in the main tank.
(14) In the above (13), the urine sensor includes the main-tank infrared sensor and a main-tank liquid level sensor for detecting a liquid level of urine contained in the main tank, the main-tank liquid level sensor includes a float floating when a water level of urine in the main tank rises and a ball valve for closing the air inlet of the airflow path by the floating of the float, and the anti-bubble net is formed cylindrically to cover the air inlet of the airflow path, the float, and the ball valve.
(15) A deodorization filter is provided to an exhaust side of the suction pump.
(16) In the above (15), a sub tank for collecting urine which has flowed into the airflow path is provided in the middle of the airflow path, the deodorization filter is installed to the lower part of the back of the body case in an oblong manner, and the sub tank is installed in the upper space of the deodorization filter on the back of the body case.

In the second aspect of the present invention, an automatic urine collecting device comprises: a main tank for collecting and containing urine received by a urine receiver; a body case for supporting the main tank; a suction pump for sucking the urine received by the urine receiver into the main tank through a urine flow path by sucking air within the main tank through an airflow path; a urine sensor for detecting urine; and a control device for outputting a control command on the basis of the detection result of the urine sensor. The automatic urine collecting device includes a sub tank for containing urine which has flowed into the airflow path in the middle of the airflow path. The urine sensor includes a main-tank sensor for detecting urine contained in the main tank and a sub-tank sensor for detecting urine in the sub tank.

In the third aspect of the present invention, an automatic urine collecting device comprises: a main tank for collecting and containing urine received by a urine receiver; a body case for supporting the main tank; a suction pump for sucking the urine received by the urine receiver into the main tank through a urine flow path by sucking air within the main tank through an airflow path; a urine sensor for detecting urine; and a control device for outputting a control command on the basis of the detection result of the urine sensor. The automatic urine collecting device includes an anti-bubble net to cover the air inlet of the airflow path opened in the main tank. The urine sensor includes; a main tank sensor for detecting urine contained in the main tank and a main-tank liquid level sensor for detecting a liquid level of urine in the main tank. The anti-bubble net is formed cylindrically to cover both the air inlet of the airflow path and the float of the main-tank liquid level sensor.

### Advantageous Effects of the Invention

According to the automatic urine collecting device of the present invention, bubbles present in the upper part of the urine contained in the main tank can be prevented from being sucked into the suction pump to improve the reliability of the suction pump.

### Brief Description of the Drawings

[Fig. 1] Fig. 1 is a block diagram showing an automatic urine collecting system using an automatic urine collecting device of one embodiment of the present invention.
[Fig. 2] Fig. 2 is a block diagram showing a control system of the automatic urine collecting system of Fig. 1.
[Fig. 3] Fig. 3 is an exploded perspective view of the automatic urine collecting device of Fig. 1.
[Fig. 4] Fig. 4 is a front perspective view of the automatic urine collecting device of Fig. 1.
[Fig. 5] Fig. 5 is a back perspective view of the automatic urine collecting device of Fig. 4.
[Fig. 6] Fig. 6 is a vertical section of the automatic urine collecting device of Fig. 4.
[Fig. 7] Fig. 7 is a front perspective view in the state where a main tank part of the automatic urine collecting device of Fig. 4 has been removed.
[Fig. 8] Fig. 8 is a planar view of an operation panel part of the automatic urine collecting device of Fig. 4.
[Fig. 9] Fig. 9 is a flowchart showing an example of operation of the automatic urine collecting device of Fig. 1.

### Best Mode for Carrying Out the Invention

Hereafter, one embodiment of the present invention is explained in reference to the drawings.

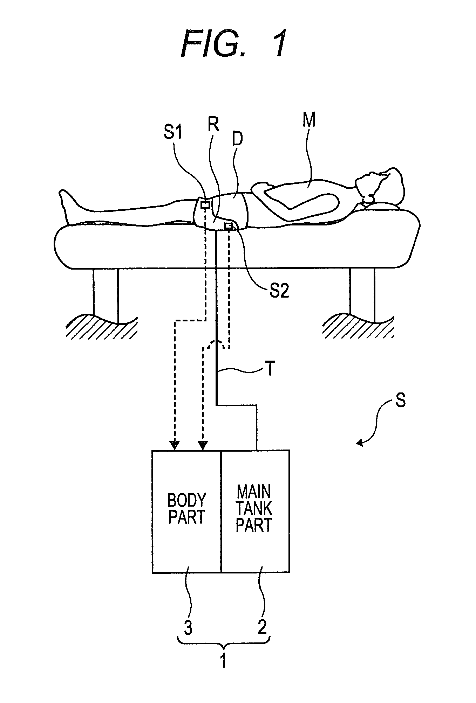

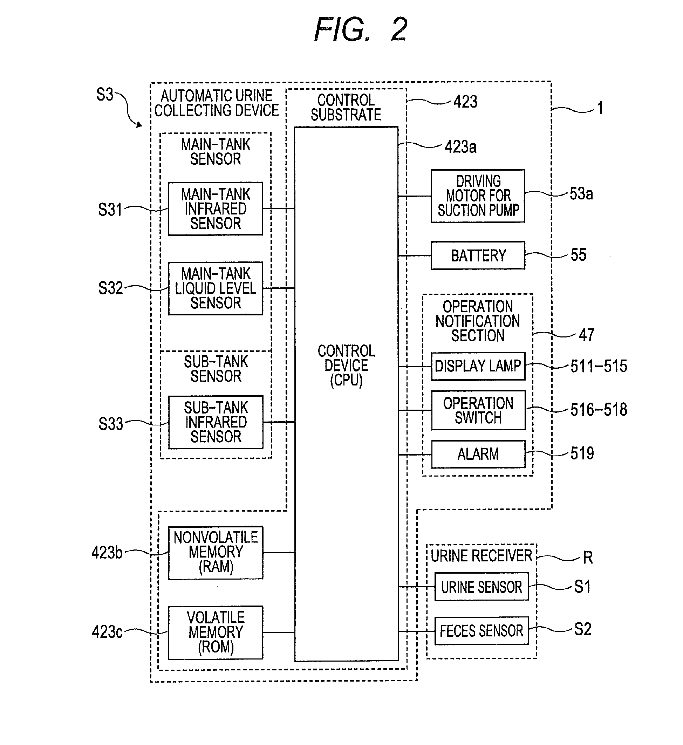

An automatic urine collecting system S using an automatic urine collecting device 1 of this embodiment is explained in reference to Figs. 1 and 2. Fig. 1 is a block diagram showing the automatic urine collecting system S using the automatic urine collecting device 1 of this embodiment. Fig. 2 is a block diagram showing a control system of the automatic urine collecting system S of Fig. 1.

As shown in Fig. 1, the automatic urine collecting system S includes: a urine receiver R mounted to a diaper (pad) D to temporarily receive urine excreted from a patient M; and the automatic urine collecting device 1 for automatically collecting urine received by the urine receiver R through a tube T. The tube T is formed of a tube made of silicone rubber or vinyl chloride having flexibility. The tube T forms part of a urine flow path for sucking the urine received by the urine receiver R into a main tank 20.

In the diaper D, a receiver urine sensor S1 for detecting urine excreted from the patient M and a receiver feces sensor S2 for detecting feces excreted from the patient M are provided. Signals detected by the receiver urine sensors S1 and receiver feces sensor S2 are transmitted to a control substrate 423 of the automatic urine collecting device 1. Both the receiver urine sensor S1 and receiver feces sensor S2 are sensors for detecting moisture and mounted in different positions to be differently used as the receiver urine sensor S1 and receiver feces sensor S2.

The automatic urine collecting device 1 includes a main tank part 2 for collecting and containing urine sent from the urine receiver R through the tube T and a body part 3 for holding the main tank part 2.

The automatic urine collecting device 1 includes the control substrate 423 as shown in Fig. 2. The control substrate 423 includes a control device 423a such as a CPU (Central Processing Unit), a nonvolatile memory 423b such as a ROM (Read Only Memory), and a volatile memory 423c such as a RAM (Random Access Memory). Programs and data required for each control are stored in the nonvolatile memory 423b. Each processing is realized when the control device 423a reads the programs of the memory for arithmetic processing.

The receiver urine sensor S1, the receiver feces sensor S2, a urine sensor S3, the nonvolatile memory 423b, the volatile memory 423c, a driving motor 53a of a suction pump 53, a battery 55, display lamps 511-515 of an operation panel part 47, control switches 516-518, an alarm 519, etc. are electrically connected to the control device 423a. The control device 423a controls various devices such as the driving motor 53a of the suction pump 53, the operation panel part 47, etc. on the basis of signals transmitted from the receiver urine sensor S1, receiver feces sensor S2, urine sensor S3, operation switches 516 to 518, etc. The urine sensor S3 includes a main-tank infrared sensor S31, a main-tank liquid level sensor S32, and a sub-tank infrared sensor S33.

In the volatile memory 423c, results etc. detected by the receiver urine sensor S1, receiver feces sensor S2, nonvolatile memory 423b, volatile memory 423c, main-tank infrared sensor S31, main-tank liquid level sensor S32, and sub-tank infrared sensor S33 are recorded. An automatic urine collecting device ID, user names, user IDs, times and dates, detection results, etc. are stored in the volatile memory 423c in association with each other.

An AC adapter (not shown) or the battery 55 supplies electrical power to the control substrate 423, the suction pump 53, and the operation panel part 47. The control substrate 423 includes an external terminal 57, and is capable of outputting operation history data (frequencies of urination, detection times of various sensors, etc.) recorded in the volatile memory 423b to external devices such as personal computers and printers.

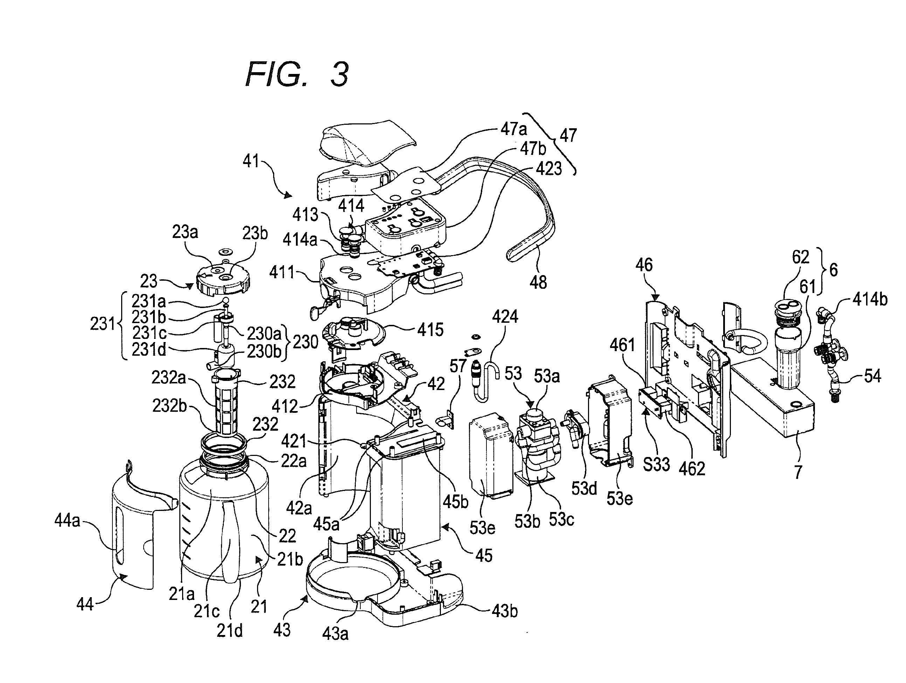

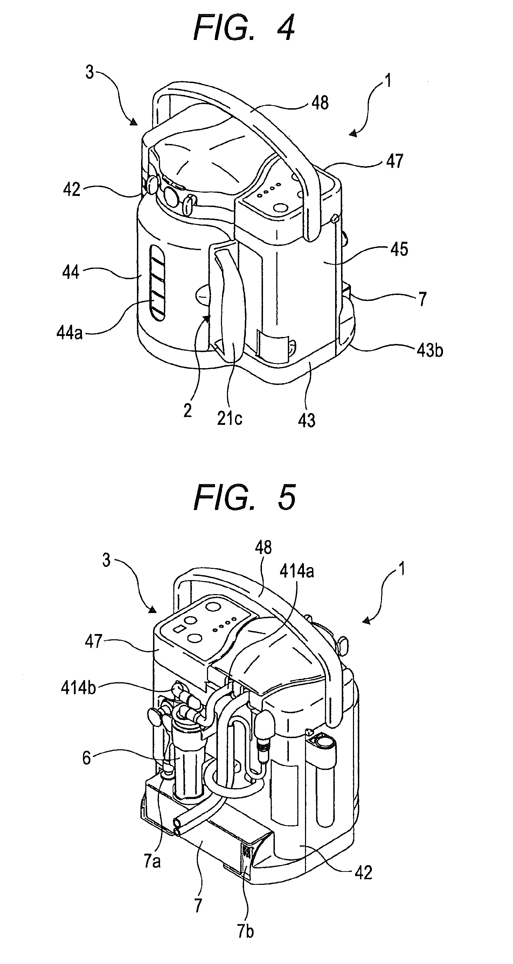

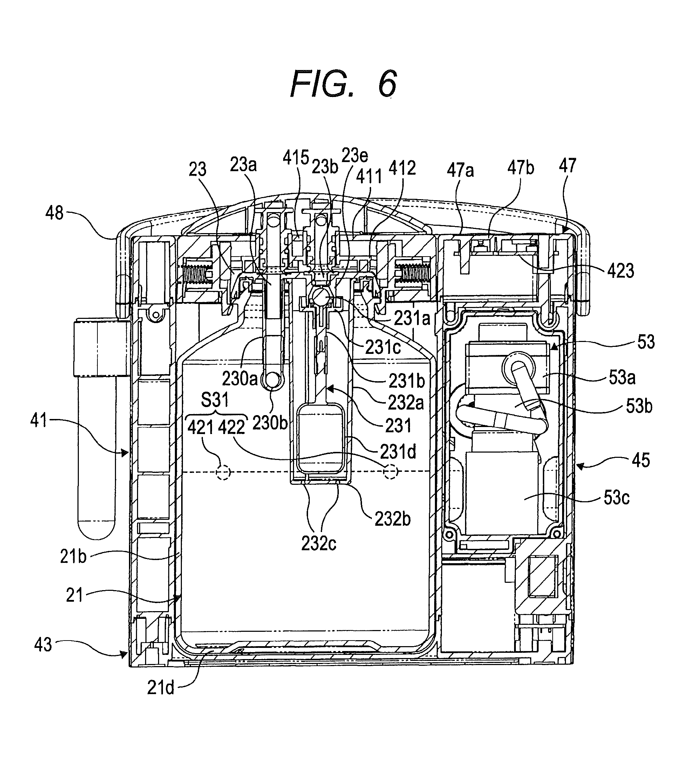

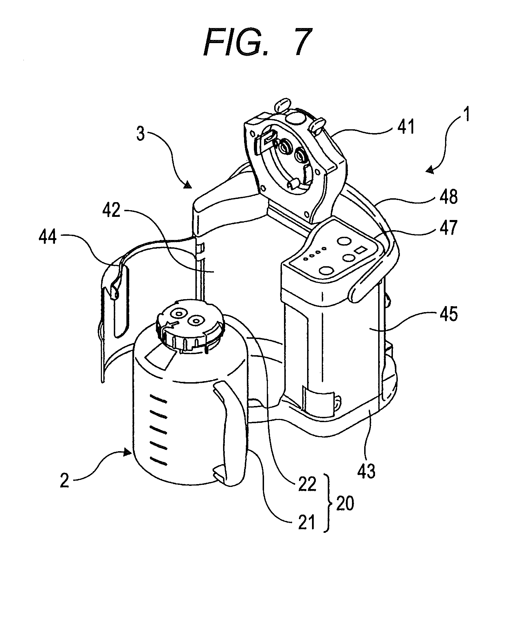

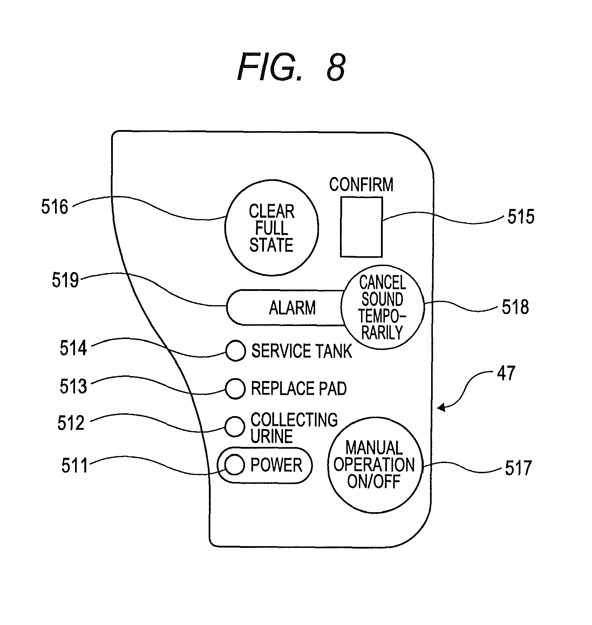

Next, the automatic urine collecting device 1 is specifically explained in reference to Figs. 3 to 8. Fig. 3 is an exploded perspective view of the automatic urine collecting device 1 of Fig. 1. Fig. 4 is a front perspective view of the automatic urine collecting device 1 of Fig. 1. Fig. 5 is a back perspective view of the automatic urine collecting device 1 of Fig. 4. Fig. 6 is a vertical section of the automatic urine collecting device 1 of Fig. 4. Fig. 7 is a front perspective view in which the main tank part 2 of the automatic urine collecting device 1 of Fig. 4 has been removed. Fig. 8 is a planar view of the operation panel part 47 of the automatic urine collecting device 1 of Fig. 4.

The main tank part 2 includes the main tank 20, a urine discharge pipe 230, suction stopping means 231, an anti-bubble net 232, etc.

The main tank 20 forms a collection container for collecting and containing urine received by the urine receiver R, and includes a main-tank body 21 having a predetermined depth and a main-tank lid 23 for sealing an opening 22 formed to an upper surface 21a of the main-tank body 21. Since the inside of the main-tank body 21 is decompressed by the suction of the suction pump 53, the main-tank body 21 has a generally closed-end cylindrical shape to obtain a predetermined strength. In this embodiment, the main-tank body 21 has the maximum capacity of 1400 mL and the effective capacity (full state capacity) of 1000 mL.

1 The upper surface 21a of the main-tank body 21 is formed in a generally hemisphere shape having the apex at its center. An opening 22 sealed by the main-tank lid 23 is formed to the apex. Thus, since the upper surface 21a is formed in the generally hemisphere shape having a predetermined angle, urine can be discarded from the opening 22 even when the main-tank body 21 is inclined at a small angle. The upper surface 21a is preferably formed to the other portions of the main-tank body 21 integrally, but may be coupled to the portions after they are molded separately.

The opening 22 is formed smaller than a diameter of a side surface 21b of the main-tank body 21. In such a structure, urine can be prevented from spilling outside for example when the main tank 20 in the full state is carried. Further, packing 232 of the main-tank lid 23, the packing being to be pressed, can be made small. Thus, since the pressure unevenness when the packing 232 is pressed is small, decompression inside the main tank 20 by the suction pump 53 becomes easy.

A thread portion 22a standing along the edge of the opening 22 has a female thread engageable with a male thread (not shown) of the inner circumference of the main-tank lid 23.

The main-tank body 21 is formed of a resin material having a weight easy to carry and a predetermined strength simultaneously, such as polycarbonate and polypropylene. The main-tank body 21 is formed of a translucent resin material through which a user can grasp a capacity of urine (hereinafter called "an amount of urine" or "urine amount") contained in the main-tank body 21 and which is transmissive to infrared light. When the main-tank body 21 is translucent, a urine amount is visible from outside in consideration for a user's privacy. Additionally, it is confirmable how the main-tank body 21 is to be inclined to discard the urine.

When the automatic urine collecting device 1 is being used, the main-tank body 21 can be covered with a tank cover 44 in consideration for user's privacy. Since a tank urine amount confirmation window 44a is provided to the center of the tank cover 44, a urine amount within the main-tank body 21 is visible through the confirmation window 44a. The confirmation window 44a is formed of a transparent member or just a hole.

A handle 21c for assisting carriage by a user is formed to the side surface 21b of the main-tank body 21. The handle 21c is used also for positioning the main tank 20 to the body part 3.

The main-tank lid 23 is a ceiled cylindrical member for sealing the opening 22 of the main-tank body 21. A male thread not shown with which a female thread of a thread portion 22b provided to the opening 22 of the main-tank body 21 is engaged is formed to the inner circumference of the main-tank lid 23.

An introduction opening 23a communicating with the tube T to introduce urine into the main-tank body 21 and an inlet opening 23b communicating with the suction pump 53 to suck air within the main tank 20 are formed to the upper surface of the main-tank lid 23. The introduction opening 23a forms part of the urine flow path, and the inlet opening 23b forms part of the airflow path.

A urine discharge pipe 230 for discharging the urine introduced from the introduction opening 23a into the main tank 20 is formed below the introduction opening 23a located in the center portion of the main tank 20. The urine discharge pipe 230 is hung from the main-tank lid 23 downward. The urine discharge pipe 230 includes a discharge conduit 230a communicating with the introduction opening 23a and extending downward and a T-shaped discharge pipe 230b communicating with the lower end of the discharge conduit 230a and branching horizontally to be opened oppositely. A width (distance between the openings on both sides) of the T-shaped discharge pipe 230b is set slightly smaller than a diameter of the opening 22 of the main tank 20.

According to such a urine discharge pipe 230, when urine is discharged from the tube T into the main tank 20 through the introduction opening 23a, a flow rate of dropping urine can be made slow and urine can drop distributing to two points toward the outer circumference in the main tank 20. Thus, scattering of urine can be eased and a height of bubbles on the upper part of the urine contained can be made low as a whole. Especially, by use of the T-shaped discharge pipe 230b, the bubbles formed on the upper part of the urine are formed in two small mount shapes. Accordingly, heights of the mounts of the bubbles can be made half or less compared to when one discharge opening is provided.

Thus, scattered urine can be prevented from being sucked from the inlet opening 23b, and the bubbles formed on the upper part of the urine can be prevented from being sucked from the inlet opening 23b. Accordingly, the possibility that the suction pump 53 and the airflow path are contaminated with the bubbles to decrease the function of the suction pump 53 can be reduced. Additionally, malodor produced from those parts can be prevented.

Below the inlet opening 23b positioned to the center portion of the main tank 20, the suction stopping means 231 for stopping suction when the main tank 20 is full of urine and the anti-bubble net 232 for preventing bubbles from being sucked from the inlet opening 23b are provided.

The suction stopping means 231 includes: a ball valve 231a for sealing the inlet opening 23b directly; a ball valve support member 231b formed of a bar-shaped member which has an upper end shaped in a saucer and supports the ball valve 231a; a container bottom lid 231c for retaining the middle of the ball valve support member 231b for free vertical movement; a float 231d connected to the lower end of the ball valve support member 231b to move vertically with a change of a liquid level; and a ball valve containing part 23e having a space inside which the ball valve 231a moves vertically. The containing part 23e is formed in a portion projecting downward from the periphery of the inlet opening 23b of the main-tank lid 23. A communication path communicating the inlet opening 23b with the inside of the main tank 20 is formed to the container bottom lid 231c or containing part 23e.

According to such suction stopping means 231, when a liquid level of urine reaches a full level (a urine amount reaches the effective capacity of 1,000 ml), the float 231d is pushed upward and the ball valve 231a seals the inlet opening 23b from below via the ball valve support member 231b. That is, in the full state, the suction is not applied further, and the introduction of urine into the main tank 20 can be stopped. That is, since urine can be prevented from being sucked from the inlet opening 23b in the full state, the possibility that the suction pump 53 and the airflow path are contaminated with urine to decrease the function of the suction pump 53 can be reduced, and production of malodor from these parts can be prevented.

The suction stopping means 231 forms part of the main-tank liquid level sensor S32. That is, the main-tank liquid level sensor includes: the float 231d for floating with rising of a liquid level of the urine in the main tank 20; the ball valve 231a for closing the inlet opening 23b of the airflow path when the float 231d floats; and detection means for detecting a current change of the driving motor 53a of the suction pump 53.

The anti-bubble net 232 is formed in a cylindrical shape to cover the inlet opening 23b and the suction stopping means 231, and is hung from the main-tank lid 23 downward. According to such anti-bubble net 232, without interfering the operation of the suction stopping means 231, bubbles can be prevented from reaching the inlet opening 23b even when the bubbles rise with rising of a liquid level of urine in the main tank 20. Thus, the possibility that the suction pump 53 and airflow path are contaminated with bubbles to decrease the function of the suction pump 53 can be reduced. Additionally, production of malodor from these parts can be prevented.

The anti-bubble net 232 includes a side surface 232a having meshes throughout and a plate-shaped bottom surface 232b having no mesh. The meshes include forty to fifty meshes per one inch. By use of the plate-shaped bottom surface 232b having no mesh, when bubbles rise with the rising of a liquid level of the urine in the main tank 20, the bubbles are pressed by the bottom surface 232b to prevent the rise of the bubbles. Thus, the bubbles can be prevented from reaching the inlet opening 23b. In this embodiment, a hole 232c having a small diameter is provided to the plate-shaped bottom surface 232b. When the anti-bubble net 232 is removed from the main-tank body 21 with the main-tank lid 23, urine in the anti-bubble net 232 can flow into the main-tank body 21 from the hole 232c.

The body part 3 includes: a body lid part 41 which is openable and is equipped with a urine flow path pipe 413 and an airflow path pipe 414; a tank housing part 42 for housing the main tank 20; a body bottom part 43 on which the main tank 20 is placed; a tank cover 44 for covering the main tank 20 in consideration for user's privacy; a pump housing part 45 which has a generally pillar shape and has mainly the suction pump 53 therein; a body back cover 46 for covering the backs of the tank housing part 42 and pump housing part 45; an operation panel part 47 having an operation panel 47a; a handle 48 used in carrying the automatic urine collecting device 1; a sub tank 6 provided in the middle of the airflow path pipe 414; and a deodorization filter 7 provided in the exhaust side of the suction pump 53. A body case includes the body lid part 41, tank housing part 42, pump housing part 45, and body bottom part 43. The tank housing part 42 includes a half-cylindrical tank housing concave surface 42a for housing the main tank 20. Part of the case forming the pump housing part 45 is shared with a portion forming the tank housing concave surface 42a of the tank housing part 42.

The body lid part 41 mainly includes an upper frame 411 and a lower frame 412 which form a base; a urine flow path pipe 413 connectable with the introduction opening 23a of the main-tank lid 23; an airflow path pipe 414 connectable with the inlet opening 23b of the main-tank lid 23; and a lid connection mechanism 415 for connecting the main-tank lid 23.

When the body lid part 41, which is openable, is being closed, connection ends of the urine flow path pipe 413 and airflow path pipe 414 are respectively connected to the introduction opening 23a and inlet opening 23b of the main-tank lid 23. When the body lid part 41 is being opened, the connection ends of the urine flow path pipe 413 and airflow path pipe 414 are separated from the introduction opening 23a and inlet opening 23b.

Unless otherwise stated hereinafter, the explanation is made on the premise that the main tank part 2 has been housed in the body part 3 and the body lid part 41 has been closed.

The upper frame 411 forms an upper wall and side wall of the body lid part 41, and has a rim formed downward from the edge of the planar upper wall around its generally entire circumference. A size of the upper frame 411 in the planar view is larger than that of the main-tank lid 23. The outer circumference of the main-tank lid 23 is located inwardly from the edge of upper frame 411. Multiple rotation shafts are formed to the back side of the upper frame 411. The multiple rotation shafts are rotatably engaged with multiple rotation shaft grooves to make the body lid part 41 openable.

The lower frame 412 forms the lower wall of the body lid part 41, and is fundamentally formed in a shape corresponding to the upper frame 411.

One end of the urine flow path pipe 413 is connected to the introduction opening 23a of the main-tank lid 23 and the other end is connected to the tube T. The urine flow path pipe 413 forms the urine flow path with the tube T, introduction opening 23a, and urine discharge pipe 230. The urine flow path is a flow path for communicating the interior space of the urine receiver R with the interior space of the main tank 20.

The airflow path pipe 414 includes a first airflow path conduit 414a arranged to the body lid part 41 and a second airflow path conduit 414b arranged to the back of the body back cover 46. The first airflow path conduit 414a has one end connected to the inlet opening 23b of the main-tank lid 23 and the other end connected to the sub-tank lid 62 of the sub tank 6. The second airflow path conduit 414b has one end connected to the sub-tank lid 62 and the other end connected to the suction pump 53. The airflow path pipe 414 forms the airflow path with the inlet opening 23b and sub tank 6. The airflow path is a flow path for communicating the interior space of the main tank 2 with the suction pump 53.

The airflow path pipe 414 and sub tank 6 are installed detachably from the body part 3 and can be easily maintained. In particular, since the components: the first airflow path conduit 414a; the second airflow path conduit 414b; a sub-tank body 61; and the sub-tank lid 62 are detachable, their insides can be cleaned easily.

The lid connection mechanism 415 connects the body lid part 41 and main-tank lid 23 to connect the urine flow path pipe 413 and airflow path pipe 414 formed to the body lid part 41 with the introduction opening 23a and inlet opening 23b formed to the main-tank lid 23.

Since the tank housing part 42 includes a half cylindrical tank housing concave surface 42a for housing the main tank 20, the overturn can be prevented when the main tank 20 loses its balance. The tank housing concave surface 42a has a shape along the side surface 21b of the main tank 20.

The main-tank infrared sensor S31, control substrate 423, and battery are housed in the tank housing part 42. The main-tank infrared sensor S31 is a main-tank urine sensor capable of detecting bubbles formed on the upper part of the urine contained in the main tank 20, and has a light emitting part 421 and a light receiving part 422 respectively arranged to both sides of the main tank 20 to oppose to one another via the main tank 20.

The light emitting part 421 and light receiving part 422 are installed to face inside the tank housing concave surface 42a from holes formed to the tank housing concave surface 42a. The light emitting part 421 and light receiving part 422 are arranged at a predetermined height to oppose to one another horizontally so that infrared light is emitted from the light emitting part 421 and received by the light receiving part 422.

When bubbles rises with the rising of a liquid level of the urine in the main tank 20 and reaches between the light emitting part 421 and light receiving part 422, infrared light emitted from the light emitting part 421 is refracted and absorbed by the bubbles and an amount of the infrared light received by the light receiving part 422 decreases. Accordingly, the main-tank infrared sensor S31 is capable of detecting the bubbles.

When a liquid level of urine reaches between the light emitting part 421 and light receiving part 422 without detecting bubbles, the infrared light emitted from the light emitting part 421 is refracted and absorbed by the urine and an amount of the infrared light received by the light receiving part 422 decreases. Accordingly, the main-tank infrared sensor S31 is capable of detecting the urine. The detection position of the urine is set to the effective capacity (full capacity) or less of the main-tank body 21 (for example, 800 mL relative to the effective capacity 1000 mL). The body bottom part 43 has a shape corresponding to a bottom surface 21d of the main tank 20 to stably install the main tank 20. An escape recess 43a corresponding to the handle 21c of the main tank 20 is formed to the upper edge of the body bottom part 43 to automatically determine an installation position of the main tank 20. The back part of the body bottom part 43 includes anti-overturn part 43b extending backward. The anti-overturn part 43b is one for preventing the overturn of the automatic urine collecting device 1.

The pump housing part 45 mainly houses the suction pump 53. The suction pump 53 is not especially limited when it is capable of sucking air. For example, it is realizable by a rotary pump. The rotary pump is a pump for sucking air within the main tank 20 by rotating a pair of rotors to have a property that it is compact and generates a great suction power and its driving sound is quiet.

The suction pump 53 includes a pump body 53a, a driving motor 53b, vibration absorber 53c, a pump connection member 53d, and a pump case 53e for unitization, which are installed replaceably. Thus, in the event that the suction pump 53 fails, it can be replaced easily.

A suction opening of the pump body 53a is connected with the airflow path pipe 414 via the pump connection member 53d. An exhaust opening of the pump body 53a is connected with the air discharging pipe 54 via the pump connection member 53d. The air discharging pipe 54 communicates with an inlet of the deodorization filter 7. Therefore, exhaust air of the suction pump 53 is introduced into the deodorization filter 7.

The driving motor 53b is arranged below the suction pump body 53a. A rubber-like vibration absorber (elastic member) 53c is arranged below the driving motor 53b. By providing the vibration absorber 53c, vibration generated from the pump body 53a and driving motor 53b is absorbed mainly by the vibration absorber 53c. The pump body 53a, driving motor 53b, vibration absorber 53c, and pump connection member 53d are housed in a pump case 53e divided into two.

The upper part of the pump housing part 45 includes an attachment part 45a for attaching the operation panel part 47 and a terminal housing portion 45b for housing the external terminal 57.

The operation panel part 47 includes the operation panel 47a, a panel base 47b, and the control substrate 423. The operation panel 47a is a seal-shaped member having a surface on which appropriate characters are applied, and is affixed to the upper surface of the panel base 47b. The control substrate 423 is arranged in the panel base 47b. The receiver urine sensor S1 and receiver feces sensor S2 are connected to the control substrate 423 via a sensor cable 424.

Control switches, display lamps, etc. corresponding to the appropriate characters applied to the operation panel 47a are provided to the control substrate 423. The panel base 47b includes an escape portion for enabling operations of the control switches and displays of the display lamps.

As shown in Fig. 8, the operation panel part 47 includes: the display lamp 511 for showing ON/OFF of power; the display lamp 512 for displaying that urine is being collected by the suction pump 53; the display lamp 513 for warning of pad replacement; the display lamp 514 for warning of service of the tank; the seven-segment display lamp 515 for identifying and displaying various states detected by the various sensors; the full-state clearing switch 516 with which a user clears an error state such as a full state; the manual operation ON/OFF switch 517 for turning the motor 53b of the suction pump 53 ON/OFF manually; and a temporal muting switch 518 for temporarily stopping an output of the alarm 519.

The sub tank 6 is connected in the middle of the airflow path pipe 414. Accordingly, in the event that urine flows into the airflow path pipe 414 from the inlet opening 23b, the urine which have flowed thereinto can be contained in the sub tank 6. The steam of urine may cause condensation inside the air inflow pipe 414 and the sub tank 6, and the condensation water also can be contained in the sub tank 6. Therefore, the possibility that the suction pump 53 is contaminated with urine and condensation water and its function is weakened can be reduced. The sub tank 6, installed detachably from the body part 3, can be removed and cleaned when contaminated with urine.

The sub tank 6 includes the elongated cylindrical sub-tank body 61 and the sub-tank lid 62 for sealing the opening formed to the upper surface of the sub-tank body 61.

The sub-tank body 61, whose inside is decompressed by the suction of the suction pump 53, is formed in a generally closed-end cylindrical shape to obtain a predetermined strength. In this embodiment, an effective capacity (full state) of the sub-tank body 61 is 50 mL. The sub-tank body 61 is formed of a translucent resin material such as one typified by polycarbonate and polypropylene, through which a user can grasp that urine has flowed into the sub-tank body 61 and which is transmissive to infrared light.

The sub-tank infrared sensor S33 is installed in the body back cover 46. The sub-tank infrared sensor S33 is a sub-tank urine sensor capable of detecting bubbles which has flowed into the sub tank 6, and includes a light emitting part 461 and a light receiving part 462 respectively arranged on both sides of the sub tank 6 to oppose to one another via the sub tank 6.

The light emitting part 461 and light receiving part 462 are installed projecting rearward from the back of the body back cover 46. The light emitting part 461 and light receiving part 462 are arranged to oppose to one another horizontally at a predetermined height so that infrared light is emitted from the light emitting part 461 and received by the light receiving part 462.

When bubbles which have flowed into the sub tank 6 from the main tank 20 are present between the light emitting part 461 and light receiving part 462, infrared light emitted from the light emitting part 461 is refracted and absorbed by the bubbles and an amount of the infrared light received by the light receiving part 462 decreases. Thus, the sub-tank infrared sensor S33 is capable of detecting bubbles of urine.

When a liquid level of urine reaches between the light emitting part 461 and light receiving part 462 without detecting bubbles, infrared light emitted from the light emitting part 461 is refracted and absorbed by the urine and an amount of the infrared light received by the light receiving part 462 decreases. Accordingly, the sub-tank infrared sensor S33 is capable of detecting the urine. The detection position of the urine is set to the effective capacity (full state) or less of the sub-tank body 61 (for example, 20 mL relative to the effective capacity of 50 mL). Even when the sub-tank infrared sensor S33 detects a liquid level of urine and stops the suction pump 53, urine may further flow into the sub tank 6 until a negative pressure in the sub tank 6 returns to the atmospheric pressure. Therefore, the detection position is preferably set to a liquid level of the full state or less.

The deodorization filter 7 is installed detachably on the anti-overturn part 43b, and the sub tank 6 is arranged in the upper space of the deodorization filter 7. In this arrangement configuration, the overall device can be made compact. The deodorization filter 7 is installed in an oblong manner in contact with the back side of the lower end of the body back cover 46 forming the body case. An inlet 7a is provided to the upper surface portion near one longitudinal side end of the deodorization filter 7, an outlet 7b is provided to the back portion near the other side end, and a deodorization member is contained therein.

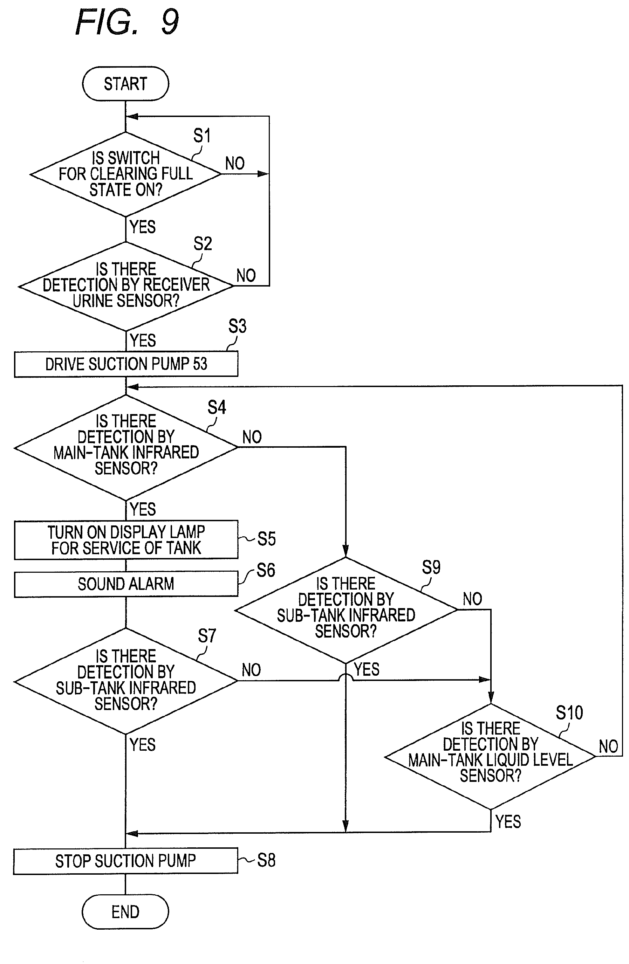

Next, processing of the main-tank infrared sensor S31, sub-tank infrared sensor S33, and main-tank liquid level sensor S32 is mainly explained as an example of processing of the automatic urine collecting device 1 of this embodiment in reference to Fig. 9. Fig. 9 is a flowchart showing an example of processing of the automatic urine collecting device 1 of Fig. 1.

When power is supplied to the automatic urine collecting device 1 and an operation of the control device 423a starts, it is determined whether the main-tank infrared sensor S31 has detected urine in the main tank 20 (Step S1). As mentioned above, detection of the urine by the main-tank infrared sensor S31 is conducted on the basis of the existence of bubbles which rise with the rising of a water level of the urine in the main tank 20.

When bubbles in the main tank 20 are detected by the main-tank infrared sensor S31 in Step S1, the display lamp 514 flashes to warn of service of the main tank 20, and warning is performed by the alarm 519 (Step S2). Thus, since a user can grasp the state of the bubbles in the main tank 20, the bubbles in the main tank 20 can be prevented from being sucked into the suction pump 53 by discarding the urine in the main tank 20. Also when a liquid level of the urine in the main tank 20 reaches a position of the main-tank infrared sensor S31 without detection of bubbles and the main-tank infrared sensor S31 detects the urine, the processing progresses to Step S2.

Then, it is determined whether the suction pump 53 is ON (Step 3). The suction pump 53 is turned ON in this Step in all the cases such as a case when the receiver urine sensor S1 is turned ON and the suction pump 53 is turned ON automatically (automatic operation) and a case when the manual operation ON/OFF switch 517 is pushed and the suction pump 53 is turned ON manually. In this automatic operation, when urine excreted by the patient M is detected by the receiver urine sensor S1, the suction pump 53 is driven only for a predetermined time. When air within the main tank 20 is sucked through the inlet opening 23b of main-tank lid 23 and the airflow path pipe 414 of the body lid part 41, the inside of the main tank 20 is decompressed. Thus, by decompressing the inside of the main tank 20, urine is sucked into and collected in the main tank 20 from the urine receiver R through the tube T, the urine flow path pipe 413 of the body lid part 41, and the introduction opening 23a of the main-tank lid 23. When a predetermined time passes after the start of the suction pump 53, the suction pump 53 stops.

When the suction pump is turned ON in Step 3, it is determined whether the sub-tank infrared sensor S33 has detected urine in the sub tank 6 (Step S4). As mentioned above, on the basis of the existence of bubbles in the sub tank 6, the sub-tank infrared sensor S33 detects the urine.

When bubbles in the sub tank 6 is detected by the sub-tank infrared sensor S33 in Step S4, the suction pump 53 stops (Step S5). Accordingly, the possibility that the suction pump 53 is contaminated with bubbles and the function of the suction pump 53 decreases can be reduced. Also when a liquid level of urine in the sub tank 6 or dew condensation water reaches the position of the sub-tank infrared sensor S33 without detection of bubbles and the sub-tank infrared sensor S33 detects the liquid level, the processing progresses to Step S5. Here, in Step S5, at the same time that the suction pump 53 stops, the display lamp 515 flashes and the alarm 519 is sounded to warn of service of the sub tank 6. At this time, specific numbers and signs are displayed on the display lamp 515 to identify a type of the warning. It is determined that a user turns ON the full-state clearing switch (Step S6). When the full-state clearing switch is turned ON, the processing returns to Step S4. The warning by the display lamp 515 and alarm 519 is continued until detection by the sub-tank infrared sensor S33 is canceled while the suction pump 53 remains stopped.

When there is no detection by the sub-tank infrared sensor S33 in step S4, it is determined whether the main-tank liquid level sensor S32 detects urine in the main tank 20 (Step S7). As mentioned above, detection of urine by main-tank liquid level sensor S32 is provided by detecting whether a liquid level of the urine in the main tank 20 reaches a full state. When the main-tank liquid level sensor S32 detects the full state in Step S7, the suction pump is stopped (Step S8). Here, in step S8, at the same time as the stop of the suction pump 53, the display lamp 515 flashes and the alarm 519 is sounded to warn of service of the main tank 20. At this time, specific numbers and signs are displayed on the display lamp 515 to identify a type of the warning. It is determined whether a user turns ON the full-state clearing switch (Step S9). When the full-state clearing switch is turned ON, the processing progresses to Step S10 to determine whether there is a detection by the main-tank infrared sensor S31 (Step S10). This is to prevent cancellation of an error while the urine contained in the main tank has not been discarded. When there is detection by the main-tank infrared sensor S31, the processing returns to Step S8. Then, the warning by the display lamp 515 and alarm 519 is continued while the suction pump 53 remains stopped until the detection by the main-tank infrared sensor S31 is canceled. When there is no detection by the main-tank infrared sensor S31 in step S10, the suction pump 53 is driven (Step S11). It is determined again whether there is the detection by the main-tank liquid level sensor S32 (Step S12). Here, the suction pump 53 is driven because in this embodiment the main-tank liquid level sensor S32 detects the full state by detecting a current change of the driving motor 53b of the suction pump 53. When there is a detection by the main-tank liquid level sensor S32 in step S12, the processing returns to Step S8 to stop the suction pump 53 and continue the warning by the display lamp 515 and alarm 519. When there is no detection by the main-tank liquid level sensor S32 in Step S12, the suction pump 53 is stopped and the processing returns to the first control (Step S13). During from Step S11 to Step S13, the suction pump 53 is driven to determine the detection by the main-tank liquid level sensor in Step S12. This duration is preferably about two seconds. The warnings generated by the display lamps 514 and 515 and alarm 519 in Step S2, S5, and S8 are canceled at the same time when the respective states are canceled. The light emitting parts 421 and 461 used in the main-tank infrared sensor S31 and the sub-tank infrared sensor S33 may emit light continuously, and may be controlled to emit light intermittently (for example, in pulses). By emitting light intermittently, a lifespan of a light emitting device can be prolonged compared to when emitting light continuously. Further, the light receiving parts 422, 462 detect an amount of light received at the time of the non light-emitting state. Accordingly, a warning can be generated when the sensors operate abnormally because an amount of infrared rays from the outside (for example, infrared rays from sunlight and room lights) is too much in comparison to the light amount emitted from the light emitting parts 421 and 461.

### [Description of Reference Numerals]

- 1: Automatic urine collecting device
- 2: Main tank part
- 3: Body part
- 6: Sub tank
- 7: Deodorization filter
- 20: Main tank
- 21: Main-tank body
- 21a: Upper surface
- 21b: Side surface
- 21c: Handle
- 21d: Bottom surface
- 22: Opening
- 23: Main-tank lid
- 23a: Introduction opening
- 23b: Inlet opening
- 23e: Ball valve containing part
- 41: Body lid part
- 42: Tank housing part
- 42a: Tank housing concave surface
- 43: Body bottom part
- 43a: Escape recess
- 43b: Anti-overturn part
- 44: Tank cover
- 45: Pump housing part
- 46: Main-body back cover
- 47: Operation panel part
- 47a: Operation panel
- 47b: Panel base
- 53: Suction pump
- 53a: Pump body
- 53b: Driving motor
- 53c: Vibration absorber
- 53d: Pump connection member
- 53e: Pump case
- 54: Air discharging pipe
- 57: External terminal
- 61: Sub-tank body
- 62: Sub-tank lid
- 230: Urine outlet pipe
- 231: Suction stopping means
- 231a: Ball valve
- 231b: Ball valve support member
- 231c: Housing base lid
- 231d: Float
- 232: Anti-bubble net
- 413: Urine flow path pipe
- 414: Airflow path pipe
- 415: Lid connection mechanism
- 421: Light emitting part
- 422: Light receiving part
- 423: Control substrate
- 423a: Control device
- 423b: Nonvolatile memory
- 423c: Volatile memory
- 511-515: Display lamp
- 516: Full-state clearing switch
- 517: Manual operation ON/OFF switch
- 518: Temporal muting switch
- 519: Alarm
- D: Diaper
- R: Urine receiver
- S1: Receiver urine sensor
- S2: Receiver feces sensor
- S31: Main-tank infrared sensor
- S32: Main-tank liquid level sensor
- S33: Sub-tank infrared sensor
- T: Tube

## Claims

1. An automatic urine collecting device comprises:
a main tank for collecting and containing urine received by a urine receiver;
a body case for supporting the main tank;
a suction pump for sucking the urine received by the urine receiver into the main tank through a urine flow path by sucking air within the main tank through an airflow path;
a urine sensor for detecting urine; and
a control device for outputting a control command on a basis of a detection result of the urine sensor,
the urine sensor including a main-tank infrared sensor capable of detecting bubbles present in an upper part of the urine contained in the main tank.

2. The automatic urine collecting device according to claim 1, comprising
notification means for warning of discard of the urine contained in the main tank, wherein
the main tank is installed to the body case detachably, and
the control device controls the notification means on a basis of a detection result of the main-tank infrared sensor.

3. The automatic urine collecting device according to claim 2, wherein
an operation panel on which a control switch and a display lamp are arranged is provided to the body case,
the notification means includes the display lamp for warning by flashing and an alarm for warning by sound, and
the control switch includes a temporal muting switch for stopping operation of the alarm.

4. The automatic urine collecting device according to claim 1, wherein
the body case has a tank housing recess,
the main tank is installed to the tank housing recess detachably and is formed of a translucent resin material transparent to infrared light, and
a light emitting part and a light receiving part respectively arranged on both sides of the main tank to oppose to one another via the main tank.

5. The automatic urine collecting device according to claim 1, wherein
the urine sensor includes the main-tank infrared sensor and a main-tank liquid level sensor for detecting urine in the main tank.

6. The automatic urine collecting device according to claim 5, wherein
the main-tank liquid level sensor includes: a float for floating when a liquid level of urine in the main tank rises; a ball valve for closing an air inlet of the airflow path by the floating of the float; and detection means for detecting a current change of a driving motor of the suction pump.

7. The automatic urine collecting device according to claim 5 or 6, comprising
notification means for warning of discard of urine contained in the main tank, wherein
the main tank is installed to the body case detachably, and
the control device controls the notification means on a basis of a detection result of the main-tank infrared sensor, and stops operation of the suction pump or controls the notification means to warn on a basis of a detection result of the main-tank liquid level sensor.

8. The automatic urine collecting device according to claim 1, wherein
a sub tank for containing urine which has flowed into an airflow path is provided in a middle of the airflow path.

9. The automatic urine collecting device according to claim 8, wherein
the urine sensor includes the main-tank infrared sensor and a sub-tank sensor for detecting urine in the sub tank.

10. The automatic urine collecting device according to claim 9, wherein
the sub-tank sensor includes a sub-tank infrared sensor capable of detecting bubbles which have flowed into the sub tank.

11. The automatic urine collecting device according to claim 10, wherein
the sub tank is formed of a translucent resin material transparent to infrared light, and the sub-tank infrared sensor includes a light emitting part and a light receiving part respectively arranged on both sides of the sub tank to oppose to one another via the sub tank.

12. The automatic urine collecting device according to claim 9, comprising
notification means for warning of discard of urine contained in the main tank, wherein
the main tank is installed to the body case detachably,
the control device controls the notification means to warn on a basis of a detection result of the main-tank infrared sensor and stops operation of the suction pump on a basis of a detection result of the sub-tank sensor.

13. The automatic urine collecting device according to claim 1, wherein
the main tank includes: a main-tank body for containing urine; and a main-tank lid installed to a central part of an upper surface of the main-tank body detachably and having openings forming part of the urine flow path and part of the airflow path adjacently; and
the urine flow path includes a T-shaped discharge pipe branching horizontally within the main tank to be opened oppositely.

14. The automatic urine collecting device according to claim 1, comprising
an anti-bubble net to cover the air inlet of the airflow path opened in the main tank.

15. The automatic urine collecting device according to claim 14, wherein
the urine sensor includes the main-tank infrared sensor and a main-tank liquid level sensor for detecting a liquid level of urine contained in the main tank, and
the main-tank liquid level sensor includes a float for floating when a liquid level of urine in the main tank rises and a ball valve for closing the air inlet of the airflow path with floating of the float, and
the anti-bubble net is formed cylindrically to cover the air inlet of the airflow path, the float, and the ball valve.

16. The automatic urine collecting device according to claim 1, wherein
a deodorization filter is provided to an exhaust side of the suction pump.

17. The automatic urine collecting device according to claim 16, wherein
a sub tank for collecting the urine which has flowed into the airflow path is provided in a middle of the airflow path,
the deodorization filter is installed to a lower part of a back of the body case in an oblong manner, and
the sub tank is installed in an upper space of the deodorization filter on the back of the body case.

18. An automatic urine collecting device comprising:
a main tank for collecting and containing urine received by a urine receiver;
a body case for supporting the main tank;
a suction pump for sucking the urine received by the urine receiver into the main tank through a urine flow path by sucking air within the main tank through an airflow path;
a urine sensor for detecting urine; and
a control device for outputting a control command on a basis of a detection result of the urine sensor,
a sub tank for containing urine which has flowed into the airflow path being provided in a middle of the airflow path, and
the urine sensor including a main-tank sensor for detecting urine contained in the main tank and a sub-tank sensor for detecting urine in the sub tank.

19. An automatic urine collecting device comprising:
a main tank for collecting and containing urine received by a urine receiver;
a body case for supporting the main tank;
a suction pump for sucking the urine received by the urine receiver into the main tank through a urine flow path by sucking air within the main tank through an airflow path;
a urine sensor for detecting urine; and
a control device for outputting a control command on a basis of a detection result of the urine sensor,
an anti-bubble net being provided to cover an air inlet of the airflow path opened in the main tank,
the urine sensor including a main-tank sensor for detecting urine contained in the main tank and a main-tank liquid level sensor for detecting a liquid level of the urine in the main tank, and
the anti-bubble net being formed cylindrically to cover both the air inlet of the airflow path and a float of the main-tank liquid level sensor.
